# EUROPEAN PATENT APPLICATION

(11) **EP 4 649 926 A1**
(43) Date of publication of application: **19.11.2025**
(21) Application number: 24176288.9
(22) Date of filing: 16.05.2024
(51) Int. Cl.: A61F 9/008, A61F 2/14

(54) **TREATMENT APPARATUS AND METHOD FOR PROVIDING A BOWMAN MEMBRANE LAYER TRANSPLANT**

(71) Applicant: SCHWIND eye-tech-solutions GmbH, 63801 Kleinostheim (DE)
(72) Inventor: ARBA MOSQUERA, Samuel, 63739 Aschaffenburg (DE); GRAUE HERNÁNDEZ, Enrique, 05370 Ciudad de México (MX)
(74) Representative: Hofstetter, Schurack & Partner

(57) **Abstract**

The invention relates to a treatment apparatus and a method for providing a Bowman membrane layer transplant. The method comprises the steps of cutting the Bowman membrane layer (18) from a donor eye (12) by an eye surgical device (22), wherein the cut is performed such that a predefined layer of stromal tissue (32) remains attached to the Bowman membrane layer (18); wherein the remaining stromal tissue (32) of the Bowman membrane layer (18) is customized depending on visual defect data of a recipient eye for the Bowman membrane layer transplant; and providing the Bowman membrane layer (18) with the customized stromal tissue (32) as a transplant for the recipient eye.

## Description

The invention relates to a method for providing a Bowman membrane layer transplant. Furthermore, the invention relates to a treatment apparatus with at least one eye surgical device that is configured to perform the method.

The Bowman layer or Bowman membrane is a layer between the epithelium and the stroma in the cornea of the eye that has no biological regenerative capacity. In particular for the treatment of progressive ectatic corneal diseases, such as Keratoglobus, Keratokonus or pellucid marginal degeneration, the transplantation of a Bowman membrane layer may stabilize the corneal changes in the eyes. For Bowman transplantation, a Bowman membrane layer is usually received from a donor eye, the epithelium of the recipients eye is removed and the donor Bowman membrane layer transplant is placed as an onlay or inlay on the recipient's stroma. The "harvesting" of a Bowman membrane layer from a donor cornea at the moment is a manual technique involving several steps that are very challenging and comprise a high failure rate. Moreover, after implanting the Bowman membrane layer low or high order aberrations may be newly introduced to the eye or existing visual defects can remain. Therefore, further treatments of the eye are often necessary to correct for the visual defects.

The objective of the present invention is to simplify and improve the transplantation of a Bowman membrane layer.

This objective is solved by the independent claims. Further advantageous embodiments are described in the dependent claims, the following description, and the figures.

The invention is based on the idea that when the Bowman membrane layer is extracted, an amount of stroma remains attached to the Bowman membrane layer, wherein the stroma may be customized in order to provide corrections for the recipient eye.

One aspect of the present invention relates to a method for providing a Bowman membrane layer transplant, wherein the method comprises the steps of cutting the Bowman membrane layer from a donor eye by an eye surgical device, wherein the cut is performed such that a predefined layer of stromal tissue remains attached to the Bowman membrane layer, wherein the remaining stromal tissue of the Bowman membrane layer is customized depending on visual defect data of a recipient eye for the Bowman membrane layer transplant, and providing the Bowman membrane layer with the customized stromal tissue as a transplant for the recipient eye.

In other words, a cut may be created encompassing the Bowman membrane layer and an amount of stroma from the donor eye. The stroma is a thick layer consisting of collagen fibers that is located below the Bowman membrane layer in the cornea of the eye. The stroma is approximately 400 to 500 µm thick and makes up around 90 percent of the total corneal thickness. The thickness of a layer of stromal tissue that remains attached to the Bowman membrane layer may be 10 percent or 20 percent of the total thickness of the stroma, in particular between 5 µm and 100 µm. The donor eye from which the Bowman membrane layer is "harvested" may be provided by an organ donor, in particular from a dead human or animal body. This means that the excision of the Bowman membrane layer may not be carried out on a living human or animal.

The cut may be performed by an eye surgical device, in particular a femtosecond eye laser or a microkeratome. During the cut, the remaining stromal tissue of the Bowman membrane layer may be customized depending on predefined visual defect data of a recipient eye, for example to correct for a visual defect. Additionally or alternatively, the remaining stromal tissue may be processed outside the donor eye after extracting the Bowman membrane layer. For example the Bowman membrane layer with the attached layer of stromal tissue may be cut in the donor eye, extracted from the donor eye and then processed, for example by a laser, in particular by an ablation laser, to customize the stromal layer in dependence of the visual defect data.

The visual defect data by which the stromal tissue is customized may comprise aberration or refraction data from the recipient, for example a value in diopters by which the recipient eye should be corrected.

After the customization, the Bowman membrane layer with the customized stromal tissue may be provided as a transplant for the recipient eye, for example as an inlay or onlay.

The advantage of the invention is that a faster preparation of the donor Bowman membrane layer and a more reliable preparation may be provided because thicker layers may be cut from a cornea in comparison to the Bowman membrane layer alone. This reduces the failure rate during preparation. Furthermore, a customized correction of aberrations, in particular higher order aberrations and/or refractive corrections, at the recipient by the attached customized layer of stromal tissue may be provided that increases the treatment satisfaction rate without sacrificing corneal tissue because of secondary treatments.

The invention also comprises embodiments by which further advantages arise.

In one embodiment the eye surgical device is a femtosecond laser, and the customization of the remaining stromal tissue is performed through a cut located posteriorly to the Bowman membrane layer of the donor eye by the femtosecond laser. A femtosecond laser may be configured to irradiate laser pulses into the tissue of the cornea, wherein cavitation bubbles are generated at the focal point of the laser beam. By aligning the cavitation bubbles, a cut may be created into the tissue of the cornea. Thus, at least one cut may be performed posterior to the Bowman membrane layer that is planned such that the layer of stromal tissue remains attached to the Bowman membrane layer. Alternatively, more cuts, in particular one anterior cut and one posterior cut may be performed by the femtosecond laser, wherein the anterior cut is on an upper side of the Bowman membrane layer and the posterior cut on a lower side of a Bowman membrane layer. In particular, the customization of the remaining stromal tissue may be performed by the femtosecond laser during the posterior cut. Therefore, properties of the layer of stromal tissue may be predefined for the femtosecond laser, for example to form the attached stroma to introduce a refractive change. The advantage of this embodiment is that a higher precision can be achieved and a faster preparation for Bowman membrane layer transplants may be provided.

In another embodiment the eye surgical device is a femtosecond laser or a micro keratome, the Bowman membrane layer with the remaining stromal tissue is cut and extracted from the donor eye, and the customization of the remaining stromal tissue is performed on the extracted Bowman membrane layer outside the donor eye. In other words, the Bowman membrane layer together with the attached layer of stromal tissue may be cut from the donor eye and then extracted. Afterwards, the customization in dependence on the visual data may be performed outside the donor eye.

In another embodiment the customization of the remaining stromal tissue is performed by an excimer laser. In particular, the customization may be performed outside the donor eye after cutting and extracting the Bowman membrane layer. The excimer laser is a gas laser that may be configured to ablate corneal tissue, in particular the attached stromal tissue. This allows to process the extracted layer of stromal tissue in a precise manner outside the cornea. Because the extracted Bowman membrane layer with the attached stromal tissue is upside down, the excimer laser may be adapted to customize the stromal tissue in a concave shape unlike the expected convex shape.

In another embodiment, the visual defect data comprises aberrations and/or a refraction of the recipient eye, wherein the aberrations and/or the refraction are compensated for by the customization of the remaining stromal tissue of the Bowman membrane layer. The aberrations and/or refractions may be measurements from the cornea or the ocular (whole eye) that are to be compensated for. In particular, the aberrations may comprise higher order aberrations such as coma, spherical aberration, and trefoil.

In another embodiment the predefined layer of stromal tissue that remains attached to the Bowman membrane layer before customization has a thickness of 5 to 50 µm. However, also a thickness of up to 100 µm may be planned for the layer of stromal tissue to provide sufficient volume for the customization.

In another embodiment, registration marks are generated in or at the customized Bowman membrane layer transplant to implant the Bowman membrane layer at a predefined location and/or a predefined orientation in the recipient eye. For example, small indentations may be created in the Bowman membrane layer transplant, for example by a laser at predefined positions. These indentations or marks may show a surgeon the required orientation of the transplant in the recipient eye. For example, the registration marks are located at the edges of the Bowman membrane layer transplant to indicate the orientation such as the top, right side, left side, and/or bottom. These registration marks may be generated in automatic manner, for example while cutting the Bowman membrane layer from the donor eye and/or during customization outside the donor eye.

Another aspect of the present invention relates to a treatment apparatus with at least one eye surgical device, wherein the treatment apparatus is configured to perform a method according to the preceding aspect. This aspect of invention provides the same advantages and variations as the method.

Further features and the advantages thereof can be taken from the descriptions of the first inventive aspect, wherein advantageous configurations of each inventive aspect are to be regarded as advantageous configurations of the respectively other inventive aspect.

Further features of the invention are apparent from the claims, the figures and the description of figures. The features and feature combinations mentioned above in the description as well as the features and feature combinations mentioned below in the description of figures and/or shown in the figures alone are usable not only in the respectively specified combination, but also in other combinations without departing from the scope of the invention. Thus, implementations are also to be considered as encompassed and disclosed by the invention, which are not explicitly shown in the figures and explained, but arise from and can be generated by separated feature combinations from the explained implementations. Implementations and feature combinations are also to be considered as disclosed, which thus do not comprise all of the features of an originally formulated independent claim. Moreover, implementations and feature combinations are to be considered as disclosed, in particular by the implementations set out above, which extend beyond or deviate from the feature combinations set out in the relations of the claims. There shows:
- Fig. 1: a schematic flow chart for a method for providing a Bowman membrane layer transplant according to an exemplary embodiment; and
- Fig. 2: a schematic flow chart for a method for providing a Bowman membrane layer transplant according to another exemplary embodiment.

In the figures, identical or functionally identical elements are provided with the same reference characters.

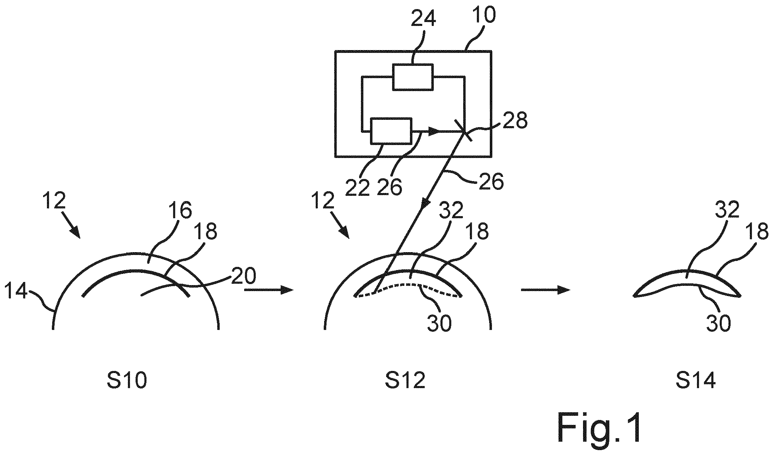

Fig. 1 shows a schematic flow chart for a method for providing a Bowman membrane layer transplant. In a step S10 a donor eye 12, in particular a cornea 14 of a donor may be provided. The donor eye 12 may be provided by an organ donor, in particular from a dead human or animal body. The cornea 14 of the donor eye 12 may comprise different layers of tissue, for example the epithelial tissue layer 16, the Bowman membrane layer 18 and the stroma 20.

In a step S12 the Bowman membrane layer 18 may be cut from the donor eye 12 by an eye surgical device 22. For example, the eye surgical device 22 may be a femtosecond laser of a treatment apparatus 10 for removing the Bowman membrane layer 18 by Photodisruption.

For example the femtosecond laser 22 of the treatment apparatus 10 may be configured to emit laser pulses in a wavelength range between 300 nm and 1400 nm, for example between 700 nm and 1200 nm, at a respective pulse duration between 1 fs and 1 ns, for example between 10 fs and 10 ps, and the repetition rate of greater than 10 kHz, for example between 100 kHz and 100 mHz.

The laser beam 26 generated by the femtosecond laser 22 can be deflected towards the donor eye 12 by a beam deflection device 28, such as for example a rotation scanner or deflection mirrors. To control the femtosecond laser 22 and/or the beam deflection device 28 the treatment apparatus 10 may comprise a control unit 24. In particular, the control unit 24 may comprise control data, such that the femtosecond laser 22 emits pulsed laser pulses in a pattern predefined by the control data. The control data may be predefined by visual defect data of a recipient eye (not shown), wherein the visual defect data may comprise visual defects of a recipient, for example aberration and/or refraction values that describe myopia, hyperopia, and/or higher order aberrations.

These visual defects of a recipient may be considered for the Bowman membrane layer transplant, in particular by performing a cut 30 below the Bowman membrane layer 18 that is customized to compensate for the visual defects of the recipient. In particular, the cut 30 is performed such that a predefined layer of stromal tissue 32 remains attached to the Bowman membrane layer 18.

In a step S14 the Bowman membrane layer 18 together with the attached and customized layer of stromal tissue 32 may be extracted from the donor eye 12, for example by removing the epithelium layer 16 or by providing a further incision cut into the cornea 14, wherein the extracted Bowman membrane layer 18 may be provided as a transplant for the recipient eye that compensates for the visual defects and stabilizes the recipient's cornea.

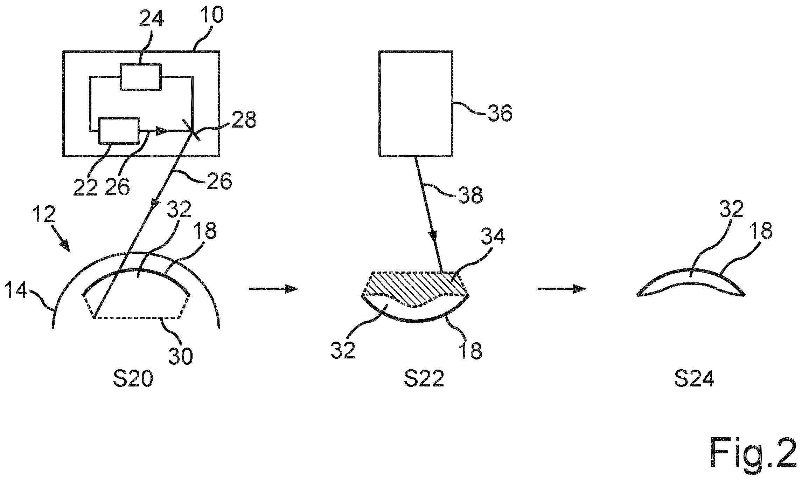

Fig. 2 shows a schematic flow chart of a method according to another exemplary embodiment. In this embodiment, a similar donor eye 12 as in step S10 may be provided from an organ donor. In this embodiment the Bowman membrane layer 18 may be cut from the cornea 14 by the eye surgical device 22, for example by the femtosecond laser 22 of a treatment apparatus 10 or a microkeratome (not shown). However, in this embodiment the cut 30 may be planned such that a predefined layer of stromal tissue 32 remains attached to the Bowman membrane layer 18 that is not yet customized to the recipient eye.

In a step S22 the Bowman membrane layer 18 with the attached stromal tissue 32 may be extracted and provided for further customization. This customization may be performed outside the donor eye 12 by an excimer laser 36. The excimer laser 36 may be a part of the treatment apparatus 10 or a separate unit. The excimer laser 36 may be configured to emit a laser beam 38 with laser pulses for example between 300 fs and 40 ns. The laser beam 38 may be configured to ablate stromal tissue 34 of the remaining stromal tissue 32, wherein the amount of ablated stromal tissue 34 may be predefined by the visual defect data of the recipient to customize the transplant for correction of a visual defect. Furthermore, registration marks may be created in the customized stromal tissue 32 that serve as orientation points for later implantation in the recipient's eye. These registration marks may be created by the ablation laser 36 during the customization. For example, the registration marks may be also created by the femtosecond laser 22 during step S20 or step S12.

After customization of the transplant by removing the stromal tissue 34 the Bowman membrane layer 18 with the customized stromal tissue 32 may be provided as a transplant for the recipient eye in a step 24.

## Claims

1. A method for providing a Bowman membrane layer transplant, wherein the method comprises the following steps:
- cutting the Bowman membrane layer (18) from a donor eye (12) by an eye surgical device (22), wherein the cut is performed such that a predefined layer of stromal tissue (32) remains attached to the Bowman membrane layer (18);
- wherein the remaining stromal tissue (32) of the Bowman membrane layer (18) is customized depending on visual defect data of a recipient eye for the Bowman membrane layer transplant; and
- providing the Bowman membrane layer (18) with the customized stromal tissue (32) as a transplant for the recipient eye.

2. The method according to claim 1, wherein the eye surgical device (22) is a femtosecond laser, and the customization of the remaining stromal tissue (32) is performed through a cut located posteriorly to the Bowman membrane layer (18) of the donor eye (12) by the femtosecond laser.

3. The method according to claim 1, wherein the eye surgical device (22) is a femtosecond laser or a microkeratome, the Bowman membrane layer (18) with the remaining stromal tissue (32) is cut and extracted from the donor eye, and the customization of the remaining stromal tissue (32) is performed on the extracted Bowman membrane layer (18) outside the donor eye (12).

4. The method according to claim 3, wherein the customization of the remaining stromal tissue (32) is performed by an excimer laser (36).

5. The method according to any one of the preceding claims, wherein the visual defect data comprises aberrations and/or a refraction of the recipient eye, wherein the aberrations and/or the refraction are compensated for by the customization of the remaining stromal tissue (32) of the Bowman membrane layer (18).

6. The method according to any one of the preceding claims, wherein the predefined layer of stromal tissue (32) that remains attached to the Bowman membrane layer before customization has a thickness of 5 to 50 µm.

7. The method according to any one of the preceding claims, wherein registration marks are generated in or at the customized Bowman membrane layer transplant, to implant the Bowman membrane layer at a predefined location and/or a predefined orientation in the recipient eye.

8. A treatment apparatus with at least one eye surgical device, wherein the treatment apparatus is configured to perform a method according to any one of the preceding claims.
